Europäisches Patentamt

European Patent Office

Office européen des brevets

(11) Veröffentlichungsnummer: **0 059 403**

A1

(12) # EUROPÄISCHE PATENTANMELDUNG

(21) Anmeldenummer: 82101311.7

(22) Anmeldetag: 20.02.82

(51) Int. Cl.³: **G 01 N 33/48**
G 01 N 1/28

(30) Priorität: 04.03.81 DE 3108133

(43) Veröffentlichungstag der Anmeldung:
08.09.82 Patentblatt 82/36

(84) Benannte Vertragsstaaten:
DE FR GB NL SE

(71) Anmelder: Battelle-Institut e.V.
Am Römerhof 35 Postfach 900160
D-6000 Frankfurt/Main 90(DE)

(72) Erfinder: Schlüter, Gert
Brunnenstrasse 41
D-6237 Liederbach(DE)

(72) Erfinder: Schuster, Wilhelm
Georg-Speyer-Strasse 64
D-6000 Frankfurt/Main(DE)

(74) Vertreter: Blum, Klaus-Dieter, Dipl.-Ing.
c/o Battelle-Institut e.V. Am Römerhof 35
D-6000 Frankfurt/Main(DE)

(54) Verfahren und Vorrichtung zur Untersuchung von Urin.

(57) Zur Untersuchung von Urin auf partikuläre Bestandteile wird der Urin unmittelbar nach der Entnahme durch ein Filter aus synthetischen, in Alkohol oder in einem Alkohol-Wasser-Gemisch auflösbaren Fasern geleitet. Das Filtermaterial mit den aufgefangenen Bestandteilen wird sofort danach in einem zellfixierenden Lösungsmittel aufgelöst, in dem es bis zur Untersuchung geschützt vor Umwelteinflüssen aufbewahrt wird.

Eine zweckmäßige Vorrichtung zur Durchführung des Verfahrens besteht aus einem Urin-Sammelgefäß (1) und einer Filterpatrone (3), die sich nach Durchlauf des Urins mit Hilfe eines Schnappverschlusses von dem Sammelgefäß trennen und mit einem Aufbewahrungsgefäß (6), in dem sich bereits das Lösungsmittel befindet, flüssigkeitsdicht verbinden läßt.

Fig. 1

0059403

390-43 - 30/80
KDB/UMA

BATTELLE - INSTITUT E.V., Frankfurt/Main

===================================================
Verfahren und Vorrichtung zur Untersuchung von Urin
===================================================

Die Erfindung bezieht sich auf ein Verfahren und eine Vorrichtung zur Untersuchung von Urin auf partikuläre Bestandteile, insbesondere auf Zellen oder kristalline Stoffe. Die Urinuntersuchung ist für die medizinische Diagnostik, auch im Rahmen von Vorsorgeuntersuchungen, von erheblicher Bedeutung.

Bei der Tumursuche im Bereich des Harntraktes hat die Urinzytologie in den letzten Jahren ständig an Bedeutung gewonnen. Dies geht sicherlich auch darauf zurück, daß unter dem Aspekt der Vorsorge die urologische Überwachung blasenturmorgefährdeter Personen, z.B. Arbeiter in der Farben- und Gummiindustrie, häufiger durchgeführt wird. Neben der Abklärung unklarer Dysurien und Hämaturien erfolgt schon heute routinemäßig die zytologische Verlaufskontrolle bei transurethral behandelten Blasenkarzinomen. Genauso wird die Strahlentherapie sowohl unter urologischen als auch unter gynäkologischen Indikationen zytodiagnostisch überwacht.

Die Urinzytologie basiert auf der Untersuchung exfolierender Zellen, die sich aus dem Gewebsverband herausgelöst haben, in den Urin abschilfern und mit dem Urin ausgeschieden werden. Die zellulären Bestandteile des Urins können sowohl von Neoplasien der Nieren, der ableitenden Harnwege als auch von den Wandungen der Blase stammen, sie können auch auf entzündliche Prozesse des gesamten Urogenitalsystems zurückgehen. Bei Frauen können auch Plattenepithelzellen aus dem Genitaltrakt und bei Männern Zellmaterial aus der Prostata mit dem Urin ausgeschieden werden.

Für die zytologische Diagnostik werden heutzutage Urinproben vom Facharzt (Urologe) oder auch vom Allgemeinpraktiker bei vorliegender Indikation gewonnen und dem Zytologen zugesandt. Die Gewinnung der Urinproben erfolgt dabei wahlweise nach folgenden Methoden:

- Spontanurin, meistens Morgenurin, wird von dem Patienten aufgefangen und in der Praxis abgeliefert,

- mit Hilfe von Kathetern werden Urinproben entnommen, wenn z.B. eine Verunreinigung der Probe mit Zellen aus dem Genitalbereich vermieden werden muß,

- in Sonderfällen wird auch über Katheterisierung Zellmaterial von Ureteren und Nierenbecken gewonnen, oder es werden Zellen durch Blasenspiegelungen entnommen.

Die Urinproben unterschiedlichen Volumens werden entweder nativ oder unter Zusatz eines Fixierungsmittels, z.B. Methanol-Essigsäure, in das Zytolabor gesandt.

Die Zellgewinnung aus der Urinprobe erfolgt wiederum nach unterschiedlichen Methoden, die vor allem von den Erfahrungen des jeweiligen Diagnoselabor abhängig sind. Die derzeit gebräuchlichen Aufbereitungsverfahren sind nachstehend aufgeführt:

- Gewinnung eines Zellsediments durch Zentrifugation. Hierbei werden Urinproben zwischen 2 und 10 ml Gesamtvolumen zentrifugiert, der Überstand abgesaugt und das Sediment zu Zellaustrichen verarbeitet. Ist im Urin wenig zelluläres Material vorhanden, müssen größere Mengen des Einsendeurins verteilt auf mehrere Zentrifugenröhrchen verarbeitet und danach die einzelnen Sedimente in einem Ausstrich vereint werden.

- In anderen Zytolabors ist es üblich, Urinproben über die Zytozentrifuge aufzuarbeiten. Nach dem Schütteln wird 0,2 ml abgezogen, zentrifugiert und dabei ein in der Zentrifuge deponierter Objektträger direkt mit den Zellen beschichtet.

- Nach der sogenannten Filtertechnik werden 10 ml Urin in einem Filterkopf gegossen, welcher z.B. mit Milliporefilter (Porengröße 5 $\mu$m) bestückt ist. Die Flüssigkeit wird durch das Filter hindurchgesaugt und die Zellen auf der Filteroberfläche abgefangen. Von dem Filter werden die Zellen auf dem Objektträger übertragen, fixiert und gefärbt.

Die Wahl der Farbstoffe für die Urinsedimentausstriche richtet sich nach den Anforderungen des jeweiligen visuellen Diagnoseverfahrens. Folgende Färbeverfahren sind gebräuchlich:

- May-Grünwald-Giemsa-Färbung
- Papanicolaou-Färbung
- Methylenblau-Färbung nach Löffler.

Alle bekannten Verfahren haben den Nachteil, daß die Diagnose der Zellen durch die lange Verweildauer im Urin erschwert wird. Im Urin treten nämlich in relativ kurzer Zeit autolytische Veränderungen der Zellen ein. Die Erhaltung des Zellzustandes unmittelbar nach deren Gewinnung ist daher für eine zuverlässige, zutreffende diagnostische Befundung von großer Wichtigkeit. In der Praxis wird dagegen die gynäkologische Zytologie heute immer mehr zu einer Einsendezytologie, weil aus verschiedenen Gründen ein hoher Anteil der zu untersuchenden Proben über den Postweg zugestellt werden muß. Dies bedeutet, daß die Urinproben vom Zeitpunkt der Entnahme bis zur präparativen Aufbereitung 1 - 2 Tage - und länger - verschiedenen Einflüssen, die die Diagnose erschweren oder verfälschen, ausgesetzt sind. Die in der Fachwelt anerkannte Forderung, eine Urinprobe sollte spätestens in 2 Stunden nach der Entnahme aufbearbeitet sein, läßt sich selbst unter günstigen Voraussetzungen mit den bisherigen Verfahren nur in Ausnahmefällen erfüllen.

Da ein guter Erhaltungszustand der Zellen Voraussetzung für eine zutreffende Diagnose ist, sind Fehlschlüsse bei der Auswertung von Urinsedimenten nach den bekannten, geschilderten Verfahren unvermeidlich. Abhängig von den Eigenschaften und der Zusammensetzung des Urins, z.B. vom pH-Wert schreitet die Zytolyse, die schon in der Blase beginnt, so lange fort, bis die Zellproben fixiert werden.

- 5 -                    0059403

Eine ausreichende Zellfixierung wird jedoch erst in einem alkoholischen Medium von mindestens 70 % erreicht, weshalb in der Praxis der Alkohol erst nach Gewinnung der Zellen, d.h. nach Abtrennen des Urins zugesetzt werden kann.

Die Urinzytologie könnte ihre prinzipiell möglichen Vorteile in der Entdeckung früher Neoplasiens, im Einsatz bei Verlaufskontrollen oder bei der Überwachung therapeutischer Maßnahmen nur dann zur Geltung bringen, wenn die geschilderten, zur Zeit noch bestehenden Nachteile überwunden wurden; dies zu erreichen, ist Aufgabe der Erfindung.

Es hat sich nun gezeigt, daß die geschilderten Probleme gelöst werden können, wenn gemäß vorliegender Erfindung bei einem Verfahren der eingangs genannten Art der Urin unmittelbar nach der Abscheidung oder Entnahme durch ein Filter aus synthetischen, in Alkohol, oder in einem Alkohol-Wasser-Gemisch auflösbaren Fasern geleitet wird, wenn anschließend, d.h. kurz danach, die Fasern in einem zellfixierenden Lösungsmittel aufgelöst werden und wenn die Probe, nämlich die durch das Filtrieren gewonnenen Zellen in diesem Lösungsmittel bis zur Untersuchung aufbewahrt werden.

Durch das erfindungsgemäße Verfahren werden auf sehr einfache Weise nahezu alle im Urin vorhandenen Zellen oder sonstigen partikulären Bestandteile aufgefangen und stehen zur weiteren Untersuchung zur Verfügung. Die sich sofort nach dem Auflösen des Filtermaterials anschließende Fixierung der Zellen garantiert, daß dem Zytologen selbst nach längerem Transport und längerer Aufbewahrungszeit noch eine optimal erhaltene Probe zur Untersuchung und zur Anfertigung der Diagnose zur Verfügung steht. Zellverluste oder Zellschädigungen, wie sie bei bisherigen Techniken (Zentrifugation, Filtertechnik) unvermeidlich sind, können nicht eintreten. In der Praxis wird es nicht mehr nötig, erneut Urinproben zu entnehmen oder Zelluntersuchungen zu wiederholen, weil selbst durch längeres Aufbewahren und unsachgemäßen Transport keine Schädigungen oder Verfälschungen mehr eintreten können. Wegen der Einfachheit und Zuverlässigkeit des erfindungsgemäßen Untersuchungsverfahrens ist dieses auch für Reihen-Vorsorgeuntersuchungen optimal geeignet.

Nach einer vorteilhaften Ausführungsart der Erfindung wird dem zellfixierenden Lösungsmittel ein Zellfarbstoff zugesetzt.

Zur Durchführung des erfindungsgemäßen Verfahrens ist eine Vorrichtung besonders gut geeignet, die im wesentlichen aus einem Sammelgefäß für den Urin und aus einer lösbar an diesem Gefäß befestigten Filterpatrone besteht, die als Filtermaterial synthetische, in Alkohol oder in einem Alkohol-Wasser-Gemisch auflösbare Fasern enthält und die nach dem Filtriervorgang, d.h. nach dem Durchlauf des Urins mit dem Aufbewahrungsgefäß flüssigkeitsdicht verbunden werden kann. Die zweite Öffnung der Filterpatrone ist zweckmäßigerweise derart dimensioniert, daß sie sich mit dem vor dem Aufsetzen der Filterpatrone abgenommenen Deckel des Aufbewahrungsgefäßes, das z.B. ein Rollrandgläschen sein kann, sich verschließen läßt. Die Verbindung zwischen Aufbewahrungsgefäß und Filterpatrone erfolgt mit Vorteil durch einen Schnappverschluß.

Weitere Merkmale, Vorteile und Anwendungsmöglichkeiten der Erfindung gehen aus der folgenden Darstellung weiterer Details anhand der beigefügten Abbildungen hervor. Es zeigen in schematischer Vereinfachung

Figur 1    eine aus Sammelgefäß und Filterpatrone bestehende
           Vorrichtung gemäß einer Ausführungsart der Erfin-
           dung und

Figur 2    ein für die Vorrichtung nach Figur 1 bestimmtes
           Aufbewahrungsgefäß.

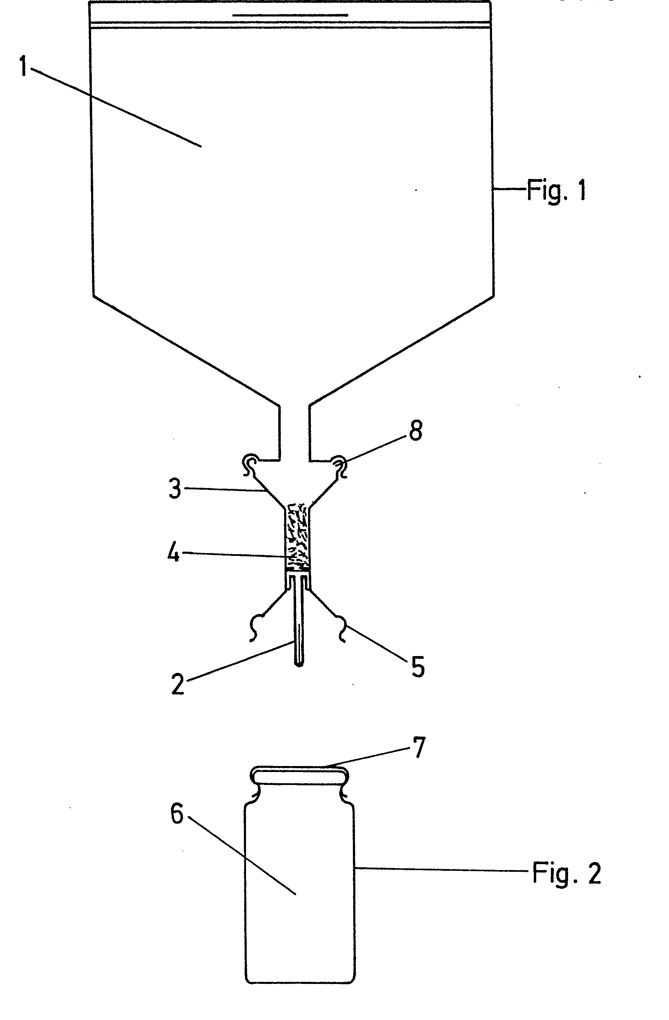

In dem in Figur 1 dargestellten Ausführungsbeispiel der
Erfindung besteht die zur Durchführung des Verfahrens geeignete Vorrichtung im wesentlichen aus einem Sammelgefäß 1,
das in Form eines Plastikbeutels zum Auffangen des Urin ausgebildet ist, und aus einer mit dem Sammelgefäß 1 flüssigkeitsdicht, aber lösbar verbundenen Filterpatrone 3. Die Verbindung zwischen dem Gefäß 1 und der Patrone 3 wird hier
über einen symbolisch angedeuteten Schnappverschluß hergestellt. Die Filterpatrone 3 enthält ein Filter 4 aus synthetischen Fasern, die in Alkohol oder in einem Alkohol-Wasser-
Gemisch löslich sind.

Zweckmäßigerweise wird zunächst der Urin in dem Sammelgefäß 1 aufgefangen, und das Gefäß dann über einen Abfluß aufgehängt. Nach Entfernen des Verschlusses 2 wird das Gefäß 1
entleert und dabei die Probe in dem Filter 4 gesammelt.
Nach Durchlauf des Urins wird dann die Filterpatrone 3 von
dem Gefäß 1 getrennt und mit dem wiederum als Schnappverschluß ausgebildeten unteren Teil 5 der Filterpatrone 3 auf
einen Aufbewahrungsbehälter 6, z.B. auf ein Rollrandgläschen,
aufgesetzt, wobei nach dem Einschnappen des federnden Teils 5
auf dem Rand des Gläschens eine ausreichend flüssigkeitsdichte Verbindung entsteht. Der vorher als Deckel 7 dienende
Verschluß des Rollrandgläschens 6 wird nun auf das entsprechend ausgebildete obere Ende 8 der Filterpatrone 3 aufgesetzt, wodurch auch an dieser Stelle ein flüssigkeitsdichter
Verschluß hergestellt wird.

In dem Aufbewahrungsgefäß 6 befand sich bereits vor Aufsetzen der Filterpatrone 3 ein zellfixierendes Lösungsmittel, nämlich Alkohol oder ein Alkohol-Wasser-Gemisch. Nachdem nun Aufbewahrungsgefäß 6, Filter 3 und Deckel 7 zu einer flüssigkeitsdichten Einheit zusammengefügt sind, wird durch mehrmaliges Schütteln oder durch Kippen der Einheit das Filtermaterial mit dem Alkohol bzw. dem Alkohol-Wasser-Gemisch in Verbindung gebracht, wodurch das Filtermaterial 4 aufgelöst und gleichzeitig die Probe, d.h. die abfiltrierten Zellen fixiert werden.

Der Filtriervorgang, das Auflösen der Fasern und das gleichzeitige Fixieren der Probe dauern nur wenige Minuten, so daß nach kurzer Zeit in dem Sammelgefäß 6 eine konzentrierte und fixierte Probe zur Verfügung steht, die in dieser Form versandt und aufbewahrt und die ohne übertriebene Eile ausgewertet werden kann. Die Weiterverarbeitung des in alkoholischer Lösung befindlichen Zellmaterials erfolgt zu einem gewünschten Zeitpunkt in üblicher Weise.

Für die mikroskopische Darstellung des abfiltrierten Materials, z.B. Zellen, ist in den meisten Fällen eine Anfärbung erforderlich. Diese läßt sich erfindungsgemäß in sehr vorteilhafter und sehr wirtschaftlicher Weise erreichen, indem man dem alkoholischen Fixier- und Lösungsmittel Farbstoffe, z.B. Methylenblau, zusetzt. Je nach Farbstoffauswahl sind zellspezifische Farbreaktionen durchführbar.

Da das erfindungsgemäße Verfahren zur Probengewinnung sich sehr einfach verwirklichen läßt, ist es nunmehr auch dem Patienten möglich, außerhalb der Praxis und ohne fachliche Hilfe diesen Vorgang selbst durchzuführen. Auch der Postversand, z.B. zum zytologischen Labor ist einfach und mit geringem Aufwand verbunden, weil selbst der Laie ohne Gefahr einer Veränderung oder Verschmutzung der Probe den Urin abfiltrieren kann, so daß die Zellprobe in konzentrierter Form vorliegt und geringes Volumen beansprucht. Da das Filtermaterial sich innerhalb der Filterpatrone befindet und bei der Durchführung des erfindungsgemäßen Verfahrens mit der Umgebung nicht in Berührung kommt, ist sichergestellt, daß die Probe nicht verschmutzen kann.

390-43 - 30/80                          23. Februar 1981
KDB/UMA


BATTELLE - INSTITUT E.V., Frankfurt/Main


Patentansprüche


1. Verfahren zur Untersuchung von Urin auf partikuläre Bestandteile, insbesondere auf Zellen, dadurch gekennzeichnet, daß der Urin unmittelbar nach der Abscheidung oder Entnahme durch ein Filter aus synthetischen, in Alkohol oder in einem Alkohol-Wasser-Gemisch auflösbaren Fasern geleitet wird, daß anschließend die Fasern in einem zellfixierenden Lösungsmittel aufgelöst werden und daß die Probe, d.h. die beim Filtrieren aufgefangenen Bestandteile in diesem Lösungsmittel bis zur Untersuchung aufbewahrt werden.

2. Verfahren nach Anspruch 1, dadurch gekennzeichnet, daß dem zellfixierendem Lösungsmittel ein Zellfarbstoff zugesetzt wird.

3. Vorrichtung zur Durchführung des Verfahrens nach Anspruch 1 oder 2, dadurch gekennzeichnet, daß diese im wesentlichen aus einem Sammelgefäß (1) für den Urin und aus einer lösbar an diesem Gefäß (1) befestigten Filterpatrone (3) besteht, die als Filtermaterial synthetische, in Alkohol oder in einem Alkohol-Wasser-Gemisch auflösbare Fasern enthält, und daß sich nach dem Filtriervorgang, d.h. nach dem Durchlauf des Urins, und nach Entfernung des Sammelgefäßes (1) eine Öffnung der Filterpatrone (3) mit einem Aufbewahrungsgefäß (6) flüssigkeitsdicht verbinden und die andere Öffnung flüssigkeitsdicht verschließen läßt.

4. Vorrichtung nach Anspruch 3, dadurch gekennzeichnet, daß sich die Filterpatrone (3) mit ihrem Auslaß auf das Aufbewahrungsgefäß (6) aufsetzen und sich der Einlaß der Filterpatrone (3) mit dem Deckel (7) des Aufbewahrungsgefäßes (6) verschließen läßt.

5. Vorrichtung nach Anspruch 3 oder 4, dadurch gekennzeichnet, daß als Aufbewahrungsgefäß (6) ein Rollrandgläschen verwendet wird und daß die Verbindung zwischen Filterpatrone (3) und dem Gefäß (6) durch einen Schnappverschluß erfolgt.

0059403

1

Fig. 1

8

3

4

2

5

7

6

Fig. 2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. ³) |
|---|---|---|---|
| Y | DE-A-2 928 790 (SARTORIUS GmbH) * Seiten 1,2; Figur 2 * | 1 | G 01 N 33/48 G 01 N 1/28 |
| Y | FR-A-2 366 554 (BATTELLE INSTITUT) * Seite 1, Zeilen 23-30; Seite 7, Zeilen 1-9 * & DE - A - 2644281 | 1 | |
| A | US-A-3 731 806 (I.B. McCORMICK) * Spalte 9, Zeile 34 - Spalte 10, Zeile 2; Figur 5 * & DE - A - 2209610 | 1 | |

RECHERCHIERTE SACHGEBIETE (Int. Cl. ³)

G 01 N 1/00
G 01 N 33/00

Der vorliegende Recherchenbericht wurde für alle Patentansprüche erstellt.

| Recherchenort | Abschlußdatum der Recherche | Prüfer |
|---|---|---|
| DEN HAAG | 02-06-1982 | ANTHONY R.G. |